(19) **Europäisches Patentamt**
**European Patent Office**
**Office européen des brevets**

(11) **EP 3 308 794 B1**

(12) **EUROPEAN PATENT SPECIFICATION**

(45) Date of publication and mention
of the grant of the patent:
**26.10.2022   Bulletin 2022/43**

(21) Application number: **16807612.3**

(22) Date of filing: **10.06.2016**

(51) International Patent Classification (IPC):
*A61P 17/02* (2006.01)    *A61K 33/30* (2006.01)
*A61K 8/27* (2006.01)    *A61Q 19/00* (2006.01)
*A61K 9/70* (2006.01)    *A61L 15/22* (2006.01)

(52) Cooperative Patent Classification (CPC):
**A61K 33/30; A61K 8/27; A61K 9/70; A61L 15/22;**
**A61P 17/02; A61Q 19/00**

(86) International application number:
**PCT/JP2016/067416**

(87) International publication number:
**WO 2016/199907 (15.12.2016 Gazette 2016/50)**

(54) **THERAPEUTIC AGENT FOR SKIN WOUND OR ROUGH SKIN**

THERAPEUTIKUM FÜR HAUTWUNDE ODER RAUE HAUT

AGENT THÉRAPEUTIQUE POUR PLAIE OU RUGOSITÉ CUTANÉE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **12.06.2015   JP 2015119548**
**04.03.2016   JP 2016042644**

(43) Date of publication of application:
**18.04.2018   Bulletin 2018/16**

(73) Proprietor: **JFE Mineral Company, Ltd.**
**Minato-ku**
**Tokyo 105-0014 (JP)**

(72) Inventors:
• **UDAGAWA, Etsurou**
**Tokyo 105-0014 (JP)**
• **ECHIZENYA, Yuko**
**Tokyo 105-0014 (JP)**
• **MIURA, Chisaki**
**Tokyo 105-0014 (JP)**
• **NAKATA, Yoshimi**
**Tokyo 105-0014 (JP)**

• **YAMAMOTO, Osamu**
**Yonezawa City**
**Yamagata 992-8510 (JP)**

(74) Representative: **Grünecker Patent- und**
**Rechtsanwälte**
**PartG mbB**
**Leopoldstraße 4**
**80802 München (DE)**

(56) References cited:
WO-A1-96/25913        WO-A1-03/082229
WO-A1-2014/026161     WO-A2-2009/019485
WO-A2-2009/129358     CN-A- 103 479 590
CN-A- 103 622 885     CN-A- 104 546 629
JP-A- 2004 269 372    JP-A- 2004 534 560
JP-A- 2005 515 191    JP-A- 2005 524 690
JP-A- 2014 511 851    US-A1- 2011 159 106
US-A1- 2014 134 272

• **DRUGS IN JAPAN : ETHICAL DRUGS 2009**
**EDITION 2008, pages 2 - 3, XP009502951**

**Description**

TECHNICAL FIELD

The present invention relates to a therapeutic agent for use in the treatment of a skin wound as well as a medical device using the therapeutic agent.

BACKGROUND ART

Zinc is the second most abundant metal in human bodies, and it has been known from a long time ago that zinc is important in healing the symptoms of dietary deficiency including dermatitis, anorexia, alopecia and impaired overall growth.

Patent Literature 1 describes "a film preparation for healing wounds, wherein a bioactive substance having activities for promoting healing of wounds is blended into a substrate of a water-soluble polymer selected from gelatin, pectin, polyvinylpyrrolidone, polyvinyl alcohol and sodium polyacrylate" in claim 1. For an adhesive used in the film preparation, an embodiment is described in which an aluminum salt and a poorly-soluble metal salt and/or a poorly-soluble metal oxide are mixed, and examples of metal salts and/or metal oxides include zinc carbonate and zinc oxide (paragraph [0026]).

Patent Literature 2 describes "a material for skin contact comprising an acid-modified, conjugated diene polymer containing at least one type of acid functional group selected from the group consisting of a carboxylic acid (salt) group, a sulfonic acid (salt) group and a phosphoric acid (salt) group in an amount of 0.01 to 5 mmol/g" in claim 1. The acid-modified, conjugated diene polymer may include, as crosslinking agents, a crosslinking agent for covalent bonding and a crosslinking agent for ionic bonding, and zinc carbonate and zinc oxide are mentioned as ionic crosslinking agents (paragraph [0022]).

However, a therapeutic agent for skin wounds or skin roughness having hydrozincite as an effective ingredient is not mentioned in any of conventional techniques.

CITATION LIST

PATENT LITERATURES

  Patent Literature 1: JP 2004-161684 A
  Patent Literature 2: JP 2005-6995 A

NON-PATENT LITERATURES

  Non-Patent Literature 1: Ken-ichi SHOFUDA, Kaoru MIYAZAKI, Kagaku to seibutsu [Chemistry and biology], Vol. 35, No. 12 (1997)
  Non-Patent Literature 2: Krüger et al., Journal of Orthopaedic Surgery and Research, 7:10 (2012)

WO 2009/129358 A2 discloses a non-lathering personal care composition in the form of an article. WO 96/25913 A1 discloses the use of monophasic zinc hydroxycarbonate as antimicrobial agent in personal care products. US 2014/134272 A1 discloses methods of enhancing wound healing. US 2011/159106 A1 discloses dermatological compositions containing an association of peroxidized lipids and zinc, and uses thereof in particular in the treatment of herpes. WO 2014/026161 A1 discloses vitamin supplement compositions for injection. JP 2004 269372 A discloses a composition for coating and applying to human body. WO 03/082229 A1 discloses a composite powder and cosmetic containing the same. JP 2005 515191 A discloses the treatment of wounds and compositions employed. JP 2004 534560 A discloses dressing material in addition to treatment solution for use with said dressing material. JP 2014 511851 A discloses an adhesive patch. JP 2005 524690 A discloses personal care compositions comprising a zinccontaining material in an aqueous surfactant composition. CN 103 622 885 A discloses a skin caring composition having function of removing acne marks, preparation and preparation method of skin caring composition. CN 104 546 629 A discloses a hydrating and whitening eye cream.

SUMMARY OF INVENTION

TECHNICAL PROBLEMS

A therapeutic agent for use in treating deep wounds and skin roughness such as a skin wound or skin roughness extending from the epidermis to the dermis is provided.

SOLUTION TO PROBLEMS

The subject matter of the invention is as set out in the appended claims.
One aspect of the invention relates to a therapeutic agent for use in the treatment of a skin wound,

wherein after a dissolution test, by a stirring method, of at least one hydrozincite-containing zinc carbonate hydroxide hydrate selected from the group consisting of hydrozincite-containing zinc carbonate hydroxide hydrate, zinc carbonate hydroxide hydrate including not less than 0.1 mass% but less than 1.5 mass% of sulfur as S as a result of at least partial substitution of carbonate ions with $SO_4^{2-}$ ions, and zinc carbonate hydroxide hydrate including not less than 0.05 mass% but less than 1 mass% of chlorine as Cl as a result of at least partial substitution of carbonate ions with $Cl^-$ ions, an amount of dissolved $Zn^{2+}$ ions is not less than 0.1 $\mu g/m^2$ and pH is not less than 7.2 but less than 8.3,

where the dissolution test is conducted with the hydrozincite-containing zinc carbonate hydroxide hydrate having a BET specific surface area of 10 to 150 $m^2/g$, a mass ratio between the hydrozincite-containing zinc carbonate hydroxide hydrate and saline being 1:50, and stirring being carried out at 500 rpm at 37°C for 3 hours by means of a rotor.

A further aspect of the invention relates to a medical device for treating a skin wound, comprising: the therapeutic agent according to the invention that is applied to a skin wound; and a wound covering material that retains the skin wound in a closed environment.
A further aspect of the invention relates to a medical set for treating a skin wound, comprising the therapeutic agent according to the invention and a wound covering material in combination.
Preferred embodiments are set forth in the subclaims.

ADVANTAGEOUS EFFECTS OF INVENTION

The therapeutic agent for use in the treatment of a skin wound according to the invention or for use in the treatment of skin roughness according to the present disclosure can regenerate skin or a hair root without inflammation. Therefore, the therapeutic agent used in the invention has a therapeutic effect on a skin wound or skin roughness extending from the epidermis but not reaching the dermis, a therapeutic effect on a skin wound or skin roughness extending from the epidermis to the dermis, or a therapeutic effect on a skin wound or skin roughness extending from the epidermis to the dermis, i.e., a full-thickness skin loss.

BRIEF DESCRIPTION OF DRAWINGS

[FIG. 1] FIG. 1 is an XRD (X-ray diffraction) chart for hydrozincite obtained by a production method of Preparation Examples of the invention.
[FIG. 2] FIG. 2 includes schematic views showing the states where a full thickness skin loss is applied with powder of Preparation Example 1 and then covered by a covering material.
[FIG. 3] FIG. 3 includes schematic views of a wound having been just produced and the wound after a while, for showing the reepithelization rate.
[FIG. 4] FIG. 4 is a view for explaining a measurement method of the reepithelization rate.
[FIG. 5] FIG. 5 includes micrographs of cross sections of skin for showing histological observation results of a wound having been just produced and the wound after a while. A is a micrograph of a cross section of stained skin after two-week treatment of the wound that was produced as described in Experimental Example 2 using Preparation Example 1; and B is an enlarged view of A.
[FIG. 6] A and B of FIG. 6 are photographs of normal skin taken at the same timing and the same magnification as those for FIG. 5, serving as the control against FIG. 5.
[FIG. 7] FIG. 7 includes schematic views for describing a healing process of a wound or a burn.

[FIG. 8] FIG. 8 is a graph showing the relationship between the pH of hydrozincite at synthesis and that after a dissolution test.

[FIG. 9] FIG. 9 is a graph showing the relationship between the pH of hydrozincite at synthesis and the amount of dissolved $Zn^{2+}$ ions after the dissolution test.

[FIG. 10] FIG. 10 is a graph showing the relationships between the pH of sulfur-containing zinc carbonate hydroxide hydrate used in the invention at synthesis and that after a dissolution test and between the pH of chlorine-containing zinc carbonate hydroxide hydrate at synthesis and that after a dissolution test.

[FIG. 11] FIG. 11 is a graph showing the relationships between the pH of sulfur-containing zinc carbonate hydroxide hydrate used in the invention at synthesis and the amount of dissolved $Zn^{2+}$ ions after the dissolution test and between the pH of chlorine-containing zinc carbonate hydroxide hydrate at synthesis and the amount of dissolved $Zn^{2+}$ ions after the dissolution test.

DESCRIPTION OF EMBODIMENTS

1. Therapeutic Agent of the Invention and the Disclosure

The therapeutic agent of the present disclosure is a therapeutic agent according to claim 1.

Zinc carbonate is carbonic acid zinc salt and is used as an abbreviation of basic zinc carbonate or zinc carbonate hydroxide. The chemical formula is $ZnCO_3$ but the composition is unstable and it is generally expressed by the typical chemical formula $2ZnCO_3 \cdot 3Zn(OH)_2 \cdot H_2O$ in the industrial field. Zinc carbonate generally represents basic zinc carbonate. It is naturally present as smithsonite. Zinc carbonate comprises zinc carbonate, zinc carbonate hydroxide and/or zinc carbonate hydroxide hydrate, is expressed by $Zn_{4-6}(CO_3)_{1-3}(OH)_{5-6} \cdot nH_2O$ ... Formula (1), where n is 0 to 6, and is sometimes expressed by the chemical formula $Zn_5(CO_3)_2(OH)_6$.

The therapeutic agent of the present disclosure is a therapeutic agent according to claim 1, wherein zinc carbonate is a therapeutic agent containing at least one selected from the group consisting of zinc carbonate, zinc carbonate hydroxide and zinc carbonate hydroxide hydrate.

The therapeutic agent for use according to the invention may be formed by using natural or commercially available zinc carbonate, zinc sulfate, zinc chloride, zinc hydroxide and zinc oxide, or by synthesizing or mixing them. Zinc oxide is mentioned as a drug in the Japanese Pharmacopoeia.

For the therapeutic agent for use according to the invention, a precipitate generated from an aqueous zinc salt solution by an alkaline precipitation process may be obtained for use. Preferably, a precipitate obtained by the reaction of $Zn^{2+}$ ions, $(CO_3)^{2-}$ ions and $OH^-$ ions in a reaction field where the pH is controlled to not less than 6.5 but less than 9.5 and more preferably not less than 7.0 but less than 9.5 in a precipitate forming reaction to be described later, is used as the therapeutic agent of the invention. More preferably, it is a therapeutic agent comprising hydrozincite-containing zinc carbonate hydroxide hydrate expressed by Formula (1):

$$Zn_{4-6}(CO_3)_{1-3}(OH)_{5-6} \cdot nH_2O \qquad (1)$$

where n is 0 to 6.

A precipitate obtained by the reaction of $Zn^{2+}$ ions, $(CO_3)^{2-}$ ions, $(SO_4)^{2-}$ ions and $OH^-$ ions in a reaction field where the pH is controlled to not less than 7.0 but less than 9.5 is used as the therapeutic agent of the invention. More preferably, it is a therapeutic agent comprising zinc carbonate hydroxide hydrate containing hydrozincite and not less than 0.1 mass% but less than 1.5 mass% of sulfur as S, expressed by Formula (2):

$$Zn_{4-6}((1-x)CO_3 + x(SO_4))_{1-3}(OH)_{5-6} \cdot nH_2O \qquad (2)$$

where n is 0 to 6, and x is 0.005 to 0.1.

A precipitate obtained by the reaction of $Zn^{2+}$ ions, $(CO_3)^{2-}$ ions, $Cl^-$ ions and $OH^-$ ions in a reaction field where the pH is controlled to not less than 7.0 but less than 9.5 is used as the therapeutic agent of the invention. More preferably, it is a therapeutic agent comprising zinc carbonate hydroxide hydrate containing hydrozincite and not less than 0.05 mass% but less than 1 mass% of chlorine as Cl, expressed by Formula (3):

$$Zn_{4-6}((1-x)CO_3 + xCl)_{1-3}(OH)_{5-6} \cdot nH_2O \qquad (3)$$

where n is 0 to 6, and x is 0.005 to 0.1.

The therapeutic agent for use according to the invention has an amount of dissolved $Zn^{2+}$ ions of not less than 0.1 $\mu g/m^2$ and a pH of not less than 7.2 but less than 8.3, and preferably an amount of dissolved $Zn^{2+}$ ions of 0.5 $\mu g/m^2$ and a pH of not less than 7.2 but less than 8.3 after a dissolution test by a stirring method, which will be described in Examples.

(Dissolution Test by Stirring Method)

The amount of dissolved $Zn^{2+}$ ions is measured in the present description, as follows: The surface areas of samples, which are produced with varying pH values at production by the same process as that for Preparation Example 2 of Examples to be described later, are measured beforehand by the BET method (BET specific surface area analyzer: High Precision, Multi-Analyte Gas Absorption Analyzer, manufactured by Quantachrome Instruments Japan G.K.). For each sample, the $Zn^{2+}$ ion concentration after stirring in saline is measured with an ICP emission spectrometer (ICPE-9000, manufactured by Shimadzu Corporation) to thereby obtain the amount of dissolved $Zn^{2+}$ ions, and then the amount of dissolved $Zn^{2+}$ ions is divided by the previously measured surface area. The mass ratio between each sample and the saline is set to 1:50, and the amount of $Zn^{2+}$ ions dissolved in the saline is measured after 3-hour stirring at 500 rpm at 37°C by means of a rotor.

2. Production Method of Therapeutic Agent for use according to the Invention

NaOH, serving as a mineralizer, is added dropwise to an aqueous solution of preferably $NaH(CO_3)$, serving as a carbonic acid source used in the alkaline precipitation process, so as to maintain the pH of the aqueous solution at preferably not less than 6.5 but less than 9.5 and more preferably not less than 7.0 but less than 9.5, and an acidic aqueous zinc solution containing zinc nitrate or the like is added dropwise to generate a precipitate, which is followed by stirring for 10 to 30 hours, its subsequent solid-liquid separation by suction filtration or centrifugation, washing with distilled water or the like, and its subsequent vacuum drying, thereby obtaining a zinc carbonate hydroxide hydrate composed primarily of hydrozincite. The obtained zinc carbonate hydroxide hydrate is not limited in particle size, and when used for a therapeutic agent, the particle size thereof can be suitably adjusted by a known method.

The carbonic acid source, the zinc source and the mineralizer, which are raw materials, are not limited to those mentioned above. Exemplary carbonic acid sources include aqueous solutions of $(NH_4)CO_3$, $Na_2CO_3$, and preferably $NaH(CO_3)$. The zinc source is selected from zinc sulfate, zinc chloride, zinc acetate and zinc nitrate. For the mineralizer, an aqueous solution of $NH_3$ or NaOH may be used. The concentration ratio (molar ratio) between carbonic acid in the carbonic acid source and zinc in an aqueous zinc source solution is preferably 2:5 for reaction, and the concentration of the aqueous zinc source solution preferably falls within the range of 0.1 to 1 M. The reaction temperature is 40°C or lower, and preferably 25°C. The obtained zinc carbonate hydroxide hydrate composed primarily of hydrozincite is a mixture of a reaction product obtained through a precipitate forming reaction between an aqueous zinc salt solution and an alkali aqueous solution, a raw material which remains unreacted, a by-product, and impurities incorporated from raw materials.

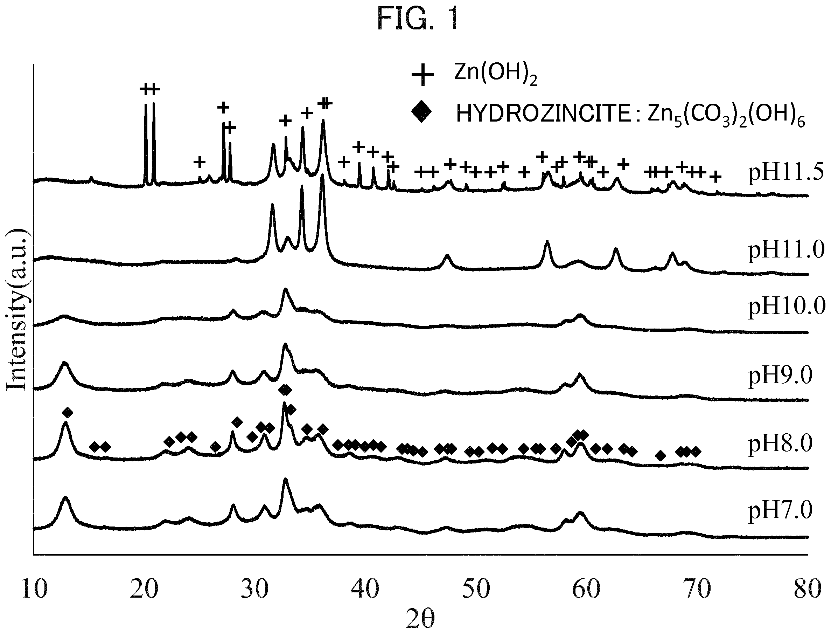

FIG. 1 shows an XRD chart of hydrozincite produced by a production method of Preparation Examples in the present invention under varying pH conditions as shown in Preparation Example 2. An XRD device used was D8 ADVANCE manufactured by Bruker Corporation.

As is seen with the cases of pH values of 7.0 to 10.0 in the chart of FIG. 1, in the pattern of the XRD diffraction peaks of zinc carbonate hydroxide hydrate containing hydrozincite, the structure of $Zn_5 (CO_3)_2 (OH)_6 \cdot nH_2O$ [Formula 1] of hydrozincite associated with the chart line at a pH of 8.0, for which peaks are marked, is dominant, and it is preferable that the a axis be 13.6 to 14.0, the b axis be 6.25 to 6.4, the c axis be 5.3 to 5.4, and $\beta$ be 95.0 to 97.5. When carbonate ions are substituted with $SO4^{2-}$ ions or $Cl^-$ ions in the structure of hydrozincite, the structure with $SO4^{2-}$ ion is expressed by Formula (2) and that with $Cl^-$ ions by Formula (3), and it is preferable that the a axis be 13.3 to 13.8, the b axis be 6.2 to 6.4, the c axis be 5.25 to 5.5, and $\beta$ be 94.9 to 97.5. When a crystal in a pattern of XRD diffraction peaks has values falling within the foregoing ranges, the effect is high as a therapeutic agent for skin wounds or skin roughness.

Table 1 shows, as the relationship between the pH at synthesis and an identified mineral phase, results of XRD diffraction of zinc carbonate hydroxide hydrate containing not less than 0.1 mass% but less than 1.5 mass% of sulfur as S that is obtained by, for instance, substituting carbonate ions with $SO_4^{2-}$ ions and zinc carbonate hydroxide hydrate containing not less than 0.05 mass% but less than 1 mass% of chlorine as Cl that is obtained by, for instance, substituting carbonate ions with $Cl^-$ ions, as produced by production methods described in Preparation Examples 3 and 4, along with the XRD diffraction results of hydrozincite shown in the XRD chart of FIG. 1.

[Table 1]

| pH at synthesis | 6 | 6.5 | 7 | 7.5 | 8 | 8.5 | 9 | 9.5 | 10 |
|---|---|---|---|---|---|---|---|---|---|
| Preparation Example 2 | - | $Zn_5(CO_3)_2(OH)_6$ Hydrozincite | $Zn_5(CO_3)_2(OH)_6$ Hydrozincite | $Zn_5(CO_3)_2(OH)_6$ Hydrozincite | $Zn_5(CO_3)_2(OH)_6$ Hydrozincite | $Zn_5(CO_3)_2(OH)_6$ Hydrozincite | $Zn_5(CO_3)_2(OH)_6$ Hydrozincite | $Zn_5(CO_3)_2(OH)_6$ Hydrozincite | $Zn_5(CO_3)_2(OH)_6$ Hydrozincite |
| Preparation Example 3 | - | $Zn_{12}(CO_3)_3(SO_4)(OH)_{16}$ Brianyoungite | $Zn_5(CO_3)_2(OH)_6$ Hydrozincite | $Zn_5(CO_3)_2(OH)_6$ Hydrozincite | $Zn_5(CO_3)_2(OH)_6$ Hydrozincite | $Zn_5(CO_3)_2(OH)_6$ Hydrozincite | $Zn_5(CO_3)_2(OH)_6$ Hydrozincite | | |
| Preparation Example 4 | - | $Zn_5(CO_3)_2(OH)_6$ Hydrozincite | $Zn_5(CO_3)_2(OH)_6$ Hydrozincite | $Zn_5(CO_3)_2(OH)_6$ Hydrozincite | $Zn_5(CO_3)_2(OH)_6$ Hydrozincite | $Zn_5(CO_3)_2(OH)_6$ Hydrozincite | $Zn_5(CO_3)_2(OH)_6$ Hydrozincite | | |

While the production conditions for obtaining hydrozincite as the main ingredient vary depending on the types and concentrations of the carbonic acid source and zinc source for use, the optimal pH condition can be found through production with varying pH conditions, the pH being kept constant in the respective conditions. The term "main ingredient" herein refers to an ingredient contained in the largest amount in a mixture, and the amount thereof is preferably not less than 80 mass% and more preferably not less than 95 mass%.

3. Production Method of Zinc Oxide

Zinc oxide can be obtained by heat-treating the precipitate obtained by the foregoing alkaline precipitation process at a temperature of 700°C or higher and preferably 1,000°C to 1,300°C. The obtained zinc oxide has excellent heat resistance, durability and safety characteristics as an inorganic antibacterial composition, and has a high antibacterial effect as an inexpensive inorganic antibacterial agent exerting an effect even in a dark place. It can be mixed in the therapeutic agent for use in the treatment of a skin wound according to the invention or in the therapeutic agent for skin roughness according to the present disclosure and used. Zinc oxide has a drying effect and antibacterial properties, and is therefore too strong. Therefore, when it is mixed in a zinc carbonate hydroxide hydrate containing hydrozincite used in the invention for use, the amount thereof is, in terms of the zinc metal amount, preferably not more than 30 mass% and more preferably not more than 10 mass% of the total zinc metal amount.

To date, many studies have been done for evaluating wound healing effects by applying zinc compounds such as $ZnSO_4$, $ZnCl_2$ and $ZnO$ to wound sites formed on experimental animals. Those compounds are substances supplying $Zn^{2+}$ ions to a wound site. Meanwhile, the fact that there is the optimum concentration of $Zn^{2+}$ ions for a wound healing effect given by $Zn^{2+}$ ions, has been reported. It is known that, while not more than 500 $\mu$mol/L of zinc ions do not exhibit toxicity to a fibroblast, a high level (not less than 15 mmol/L) of zinc ions lead to increased inflammatory cell infiltration in skin to remarkably delay reepithelization.

The pH of the body fluid composed primarily of saline is maintained at about 7.4 to about 7.5, and when, for example, basic zinc carbonate composed primarily of hydrozincite used in the invention is dissolved in saline, the pH is expected to vary in accordance with formation and dissolution of $Zn^{2+}$ ions. In addition, by figuring out two factors including a pH environment supplying $Zn^{2+}$ ions and/or $(CO_3)^{2-}$ ions and the supply of $OH^-$ ions for efficiently activating matrix metalloproteinase (MMP) enzymes that decompose matrix proteins by the aid of OHions of water molecules, further healing proceeds, and tissue hindering healing such as a crust is not formed, and a scar can be prevented from remaining, so that improved QOL (quality of life) of patients can be expected.

It is known that cells actively grow and migrate in a wound healing process. When a cell migrates within a tissue or between tissues, it is necessary to locally break existing extracellular matrix. At the same time, formation of new extracellular matrix proceeds in order to remodel tissue at a wound site. In those processes, various proteolytic enzymes are involved.

The activation balance between matrix metalloproteinases (MMPs) and tissue inhibitors of metalloproteinases (TIMPs) is related to both of normal and pathologic events such as wound healing, tissue repair, vascularization, infiltration, tumorigenesis and tumor metastasis.

The MMPs are enzymes degrading extracellular collagen and are synthesized by connective tissue and cells present in connective tissue. The MMP has a zinc ion in its center, and a binding site of $Zn^{2+}$ ion is present at an active site. Epidermal regeneration is achieved when epidermal cells migrate from a wound edge or skin appendages (e.g., hair roots, sweat glands). Thus, bonding between $Zn^{2+}$ ions from a zinc compound and the MMPs triggers destruction of extracellular matrix, thereby promoting migration of epidermal cells.

The TIMPs are enzymes produced by fibroblasts, endothelial cells and the like, and having an inhibitory effect against the MMPs. The MMPs and the TIMPs form a 1:1 complex to inhibit collagen degradation by the MMPs. Owing to this mechanism, it is possible to suppress specific cleavage of helix sites of type I, type II and type III collagens by the MMPs, thereby promoting fibrosis at a wound site and increasing the amount of collagen in regenerated tissue.

From the above, it is conceivable that the therapeutic agent for use in the treatment of a skin wound according to the invention or for use in the treatment of skin roughness according to the present disclosure can promote migration of cells by suitably supplying zinc ions to a wound site, thereby increasing accumulation of collagen and promoting healing of the wound.

In addition, by figuring out a pH environment supplying zinc ions and/or carbonate ions, further healing proceeds, and a tissue hindering healing such as a crust is not formed, and a scar can be prevented from remaining, so that improved QOL (quality of life) of patients can be expected.

4. The therapeutic agent used in the invention can be used as an additive for pharmaceutical compositions and an additive for cosmetics.

(1) The therapeutic agent used in the invention has less drying effect and antibacterial properties compared to zinc oxide, and is useful as an additive. It contains, as an effective ingredient, hydrozincite-containing zinc carbonate hydroxide hydrate, hydrozincite-containing zinc carbonate hydroxide hydrate including not less than 0.1 mass% but less than 1.5 mass% of sulfur as S as a result of, for instance, substitution of carbonate ions with $SO_4^{2-}$ ions, or hydrozincite-containing zinc carbonate hydroxide hydrate including not less than 0.05 mass% but less than 1 mass% of chlorine as Cl as a result of, for instance, substitution of carbonate ions with $Cl^-$ ions, in an amount of dissolved $Zn^{2+}$ ions per surface area of not less than 0.1 $\mu g/m^2$ and at a pH of not less than 7.2 but less than 8.3 after a dissolution test by a stirring method, and is favorable as an additive used in pharmaceutical compositions or an additive for cosmetics.

(2) Besides, it contains, as effective ingredients, hydrozincite-containing zinc carbonate hydroxide hydrate, zinc carbonate hydroxide hydrate including not less than 0.1 mass% but less than 1.5 mass% of sulfur as S as a result of at least partial substitution of carbonate ions with $SO_4^{2-}$ ions, and zinc carbonate hydroxide hydrate including not less than 0.05 mass% but less than 1 mass% of chlorine as Cl as a result of at least partial substitution of carbonate ions with $Cl^-$ ions, in an amount of dissolved $Zn^{2+}$ ions per surface area of not less than 0.1 $\mu g/m^2$ and at a pH of not less than 7.2 but less than 8.3 after a dissolution test by a stirring method, and is favorable as an additive for cosmetics.

The dissolution test is conducted under the following conditions: hydrozincite-containing zinc carbonate hydroxide hydrate, zinc carbonate hydroxide hydrate including not less than 0.1 mass% but less than 1.5 mass% of sulfur as S as a result of at least partial substitution of carbonate ions with $SO_4^{2-}$ ions, and zinc carbonate hydroxide hydrate including not less than 0.05 mass% but less than 1 mass% of chlorine as Cl as a result of at least partial substitution of carbonate ions with $Cl^-$ ions each have a BET specific surface area of 10 to 150 $m^2/g$; the mass ratio of each of hydrozincite-containing zinc carbonate hydroxide hydrate, zinc carbonate hydroxide hydrate including not less than 0.1 mass% but less than 1.5 mass% of sulfur as S as a result of at least partial substitution of carbonate ions with $SO_4^{2-}$ ions, and zinc carbonate hydroxide hydrate including not less than 0.05 mass% but less than 1 mass% of chlorine as Cl as a result of at least partial substitution of carbonate ions with $Cl^-$ ions to saline is 1:50; and stirring is carried out at 500 rpm at 37°C for 3 hours by means of a rotor.

5. Therapeutic Agent for Skin Wounds or Skin Roughness and Pharmaceutically Acceptable Carrier Thereof

The therapeutic agent used in the invention may be used as a therapeutic agent for skin wounds or skin roughness and may optionally include a pharmaceutically acceptable carrier. Exemplary carriers include organic solvents and inorganic solvents, more specifically, water, saline, alcohols, polyhydric alcohols, and mixtures thereof. This therapeutic agent may be processed by adding a thickener or the like so as to have a gel or paste form, thereby improving handleability.

Exemplary carriers include hydrocarbons such as $\alpha$-olefin oligomer, paraffin wax, ceresin and microcrystalline wax; animal and vegetable oils such as persic oil, olive oil, beef tallow and mink oil; synthetic esters such as cetyl octanoate, isopropyl myristate and cetyl palmitate; natural animal and vegetable waxes such as jojoba oil, carnauba wax, candelilla wax, Japan wax and bees wax; sorbitan stearate; polyoxyethylene glyceryl tristearate; polyoxyethylene lauryl ether; decaglyceryl trioleate; sucrose monolaurate ester; and silicone oils and derivatives thereof such as dimethylpolysiloxane and methyl phenyl polysiloxane.

Further examples that may be used include fluororesins such as perfluoropolyether; alcohols such as ethanol, 1,3-butylene glycol, propylene glycol and diglycerine; carrageenan, xanthan gum, carboxymethyl cellulose sodium, collagen, elastin, silk, cellulose, lactoferrin and other proteins and hydrolysates thereof; and powders of anhydrous silicate, nylon powder, polyalkyl acrylate, alumina and iron oxide.

Still other examples include ultraviolet absorbers, vitamins, urea, sea-water dried products, anti-inflammatory agents, amino acids and derivatives thereof, lecithin, coloring agents, fragrances, preservatives, and oils such as eggyolk oil, macadamia nut oil, cottonseed oil, avocado oil, coconut oil, palm oil, palm kernel oil, corn oil, peanut oil, beef tallow and carnauba wax.

Still other examples include bees wax, liquid paraffin, lanolin, squalane, stearic acid, laurates, myristates, isostearyl alcohol, purified water, electrolyzed water and ethyl alcohol. That is, any substance blended in both of cosmetics and quasi drugs in general is usable as a carrier of the therapeutic agent for use in the treatment of a skin wound according to the invention or for use in the treatment of skin roughness according to the present disclosure. A carrier is not necessarily used.

For instance, other optional ingredients that may be added to the therapeutic agent for use in the treatment of a skin wound according to the invention or for use in the treatment of skin roughness according to the present disclosure are selected depending on the substance actually added to a cosmetic or a quasi drug. Although not strictly distinguishable, exemplary humectants include glycerol, sorbitol, polyethylene glycol, pyrrolidone carboxylic acid and its salt, collagen, 1,3-butylene glycol, hyaluronic acid and its salt, chondroitin sulfate and its salt, and xanthan gum.

Exemplary antioxidants include ascorbic acid, $\alpha$-tocopherol, dibutylhydroxytoluene and p-hydroxyanisole. Exemplary surfactants include sodium stearyl sulfate, diethanolamine cetyl sulfate, polyethylene glycol monostearate, ethylene glycol monostearate, polyoxyethylene hydrogenated castor oil, soybean lysophospholipid liquid, and polyoxyethylene sorbitan monooleate.

Exemplary preservatives include phenoxyethanol, ethylparaben, butylparaben, and inorganic pigments such as zinc oxide.

Exemplary antiphlogistic agents include glycyrrhizic acid derivatives, salicylic acid derivatives, hinokitiol, zinc oxide and allantoin.

Exemplary whitening agents include placental extract, glutathione, saxifraga sarmentosa extract, ascorbic acid derivatives and arbutin.

Exemplary blood circulation promoting agents include $\gamma$-oryzanol and dextran sulfate sodium.

Exemplary antiseborrheic agents include sulfur and thianthol.

Exemplary thickeners include carboxyvinyl polymer.

Exemplary pH adjusters include lactic acid, citric acid, malic acid, glycolic acid, sodium hydroxide and hydrotalcite.

The concentration of the therapeutic agent is not particularly limited, and as an example, the zinc concentration is 0.000001 g/L to 10 g/L in terms of zinc metal with respect to the total amount of the therapeutic agent. The solid content in the therapeutic agent is preferably 0.00000173 g/L to 17.3 g/L, and more preferably 0.000173 g/L to 1.73 g/L in terms of zinc carbonate hydroxide hydrate or hydrozincite ($Zn_5 (CO_3)_2 (OH)_6 \cdot H_2O$). The concentration within the foregoing ranges leads to a high therapeutic effect.

When the therapeutic agent is dissolved in saline for use with the ratio between the therapeutic agent and the saline being 0.1 g/L to 100 g/L, the therapeutic agent being the one expressed by one of Formulae (1) to (3) above, and n = 0 (anhydrous), the therapeutic agent for skin wounds or skin roughness having a zinc concentration of 45 mass% to 75 mass% in terms of zinc metal with respect to the total amount of the therapeutic agent and a zinc concentration in the saline of 0.045 g/L to 75 g/L is preferred.

6. Medical Device for Treating Skin Wounds or Skin Roughness and Wound Covering Materials

The therapeutic agent used in the invention is effective at treating skin wounds or skin roughness extending from the epidermis to the dermis without limitation. The therapeutic agent used in the invention may comprise a medical device for treating skin wounds or skin roughness that is applied to a skin wound or skin roughness and retains the skin wound or the skin roughness in a closed environment with a wound covering material. The expression "applied to a skin wound or skin roughness" may refer to direct application to a skin wound or skin roughness or application to skin in an area around a skin wound or skin roughness. A wound covering material is a medical device that retains a skin wound or skin roughness in a closed environment. A medical device in another embodiment may be configured such that the therapeutic agent for use according to the invention is applied, contained or attached on or in a wound covering material and the wound covering material on or in which the therapeutic agent for use according to f the invention is applied, contained or attached retains a skin wound or skin roughness in a closed environment.

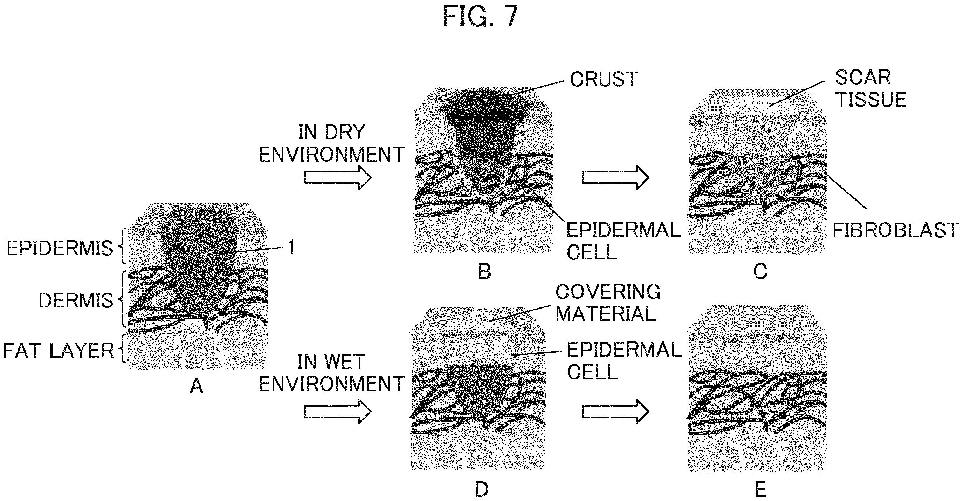

The therapeutic agent for use in the treatment of a skin wound according to the invention or for treating a skin roughness according to the present disclosure and the medical device for treating a skin wound according to the invention (hereinafter sometimes called "medical device of the invention") or for treating skin roughness according to the present disclosure are capable of treating, without limitation, skin wounds or skin roughness that are skin losses of the epidermis and the dermis (i.e., full-thickness skin losses). This is described with reference to the schematic views of FIG. 7.

A of FIG. 7 is a schematic view showing the cross section of skin, with a skin wound or a rough skin part that extends from the epidermis to the dermis being designated by 1. In a dry environment in which the wound is dried, the therapeutic agent used in the invention is applied to an inner surface of the wound, and a crust like a scab is formed or the upper surface of the wound is protected by, for instance, a breathable adhesive bandage. As shown in B, epidermal cells occur in the inner surface of the wound with healing of the wound. When the healing further proceeds, as shown in C, fibroblasts cover the wound, while scar tissue develops at the upper surface of the wound, and thus the wound heals.

In a wet environment in which exudate is produced and the wound is wet, which is shown in D, the upper part of the wound is covered by a water-absorbing wound covering material to be described later, and the therapeutic agent used in the invention is applied to the top surface of the wound. In such an environment, the medical device of the invention can provide the optimal environment for proliferation of granulation tissue and transfer of epithelial cells. As shown in D, epidermal cells occur in the top or inner surface of the wound with healing of the wound. When the healing further proceeds, as shown in E, fibroblasts cover the wound, while skin tissue develops at the upper surface of the wound, and thus the wound heals.

Wound Covering Material

In healing of skin wounds or skin roughness, there are two cases of healing in a dry environment and healing in a wet environment. In healing in a dry environment, although no wound covering material is applied in some cases, generally a breathable wound covering material is applied to protect a wound surface or a rough skin part. There are wound covering materials of gauze type, bandage type and breathable film type.

The therapeutic agent for use in the treatment of a skin wound according to the invention or for use in the treatment of skin roughness according to the present disclosure can provide a closed environment using a wound covering material to retain a wound in a further proper wet environment, and is therefore effective at treatment. When the therapeutic agent used in the invention that is an inorganic material and a wound covering material that is an organic material are combined, the organic and inorganic materials are hybridized, leading to a great synergy.

In use of the therapeutic agent for use according to the invention, a liquid form may be employed with the use of saline or the like as the solvent and, when a wound has exudate and is wet, a powder dosage form may be employed. Viscous forms and gels between the above forms may be employed, if necessary. The therapeutic agent for use in the treatment of a skin wound according to the invention or for use in the treatment of skin roughness according to the present disclosure can provide a closed environment using a wound covering material and, in addition, be made to take a liquid or powder dosage form so as to maintain a wet state suitable for each wound, and is therefore effective at treatment in a wet environment. In either of liquid and powder dosage forms, when the therapeutic agent used in the invention is used along with a wound covering material, tissue hindering healing such as a crust is not formed, and a scar can be prevented from remaining, so that improved QOL (quality of life) of patients can be expected.

Healing of wounds can be promoted by providing a wet environment using the following wound covering materials:

1) Polyurethane Film Dressing

Examples include, by the trade names, Tegaderm (manufactured by 3M), Opsite Wound (manufactured by Smith & Nephew plc), I V3000 (manufactured by Smith & Nephew plc) and Bioclusive (manufactured by Johnson & Johnson). They are polyurethane film dressings formed of transparent films each having one side constituting an adhesive surface and each of which allows steam and oxygen to permeate therethrough so that the inside is not steamed. They are used for the purposes of protection of non-bleeding wound surfaces, shallow pressure ulcers (only redness) or blisters, and prevention of pressure ulcers, and are useful in sealing alginate covering materials and hydrogels, which will be described later.

2) Hydrocolloid Dressing

Examples include, by the trade names, Duoactive (manufactured by ConvaTec Inc.), Comfeel (manufactured by Coloplast Corp), Tegasorb (manufactured by 3M) and Absocure (manufactured by Nitoms, Inc.). They are of sheet type with the outer side constituting a waterproof layer and the inner side constituting an adhesive surface containing hydrophilic colloid particles. Their sizes and shapes vary. For the thickness, some dressings are formed using polyurethane foam to provide cushioning properties and are thus thick (Duoactive, CGF), while some are very thin and flexible (manufactured by Duoactive ET). While those of sheet type are used in general, those of paste type (manufactured by Comfeel Paste) and those of granular type (Duoactive) are used for pressure ulcers with a recess or a sinus. Owing to the outer waterproof layer that blocks the affected part from the air, it is possible to, especially in the case of a pressure ulcer in the sacral region, protect a wound surface from urine and fecal incontinence. The hydrophilic colloid particles on the inner side absorbs exudate and thereby become wet gel, thus providing a wet environment. Note that oxygen in the air is blocked. Since colloid particles on a wound surface gelate, the dressing does not adhere to the wound surface. For fresh wounds, there is Duoactive that is thin.

3) Polyurethane Foam Dressing

Examples include, by the trade name, Hydrosite (manufactured by Smith & Nephew plc). The outermost side is formed from a polyurethane film impervious to water, the innermost side is formed of non-adherent thin polyurethane, and a thick layer of hydrophilic absorbent foam is sandwiched therebetween. The middle layer has high water absorbing properties, absorbs exudate and holds a moderate amount of moisture, thereby maintaining a wet environment of a wound surface. Therefore, they are used for a heavily exuding wound surface. If used for a heavily exuding pressure ulcer, hydrocolloid quickly dissolves, and surrounding skin may excessively macerate. In such a case, polyurethane is more convenient. In addition, since the dressing itself does not dissolve and does not remain on the wound surface, handling is extremely easy.

They are usable for all types of skin loss wounds if they are fresh wounds, and the application to fingertip wounds is preferable. A fingertip area is a portion easily hit even in normal times, and a hit against a wound site in a fingertip wound is accompanied with severe pain. When a fingertip area is covered by Hydrosite, however, since the dressing itself is thick and has cushioning properties, the impact of hitting is reduced, which is advantageous. Basically, the dressings themselves are non-adherent and need to be fixed with an adhesive bandage or the like. However, when the patient is an elderly person having extremely fragile skin and the skin is likely to be torn as if "wet Shoji paper is torn," only a bandage may be wrapped without fixation with an adhesive bandage.

4) Alginate Covering Material

Examples include, by the trade names, Kaltostat (manufactured by ConvaTec Inc.), Sorbsan (manufactured by Alcare Co., Ltd.), Algoderm (manufactured by Medicon, Inc.) and Kurabio (manufactured by Kuraray Co., Ltd.). They are formed of nonwoven fabric obtained by processing alginate (calcium salt for Sorbsan and Algoderm; a mixture of calcium salt and sodium salt for Kaltostat) extracted from kelp, which is a seaweed, into a fibrous form. Alginate absorbs 15 to 20 times as much moisture as its own weight, and gelates upon absorption of moisture containing sodium ions such as exudate. This gel maintains a wet environment of a wound surface. In addition, they have an extremely strong hemostatic effect. This is exerted upon emission of calcium ions at gelating. They are usually sealed with a film dressing for use. Among several types of products, Kaltostat has a low gelation rate and is hardly broken owing to its thick and hard fibers, whereas Sorbsan has thin fibers and therefore becomes soft gel and is easily broken; however, there is no great difference therebetween in actual use. Bleeding that may occur when a wound surface is brushed in the case of, for instance, a contusion, can be treated with an alginate covering material. Because of the foregoing characteristics, this type of covering materials are preferably used for "skin loss wounds accompanied by bleeding."

5) Hydrogel Dressing

Examples include, by the trade names, Gelibalm (manufactured by Taketora Holdings Co., Ltd.), Nu-Gel (manufactured by Johnson & Johnson), Intrasite (manufactured by Smith & Nephew plc), GranuGEL (manufactured by ConvaTec Inc.) and Clearsite (manufactured by Nippon Sigmax Co., Ltd.). They look like "transparent ointment" at first glance, but hydrophilic polymer molecules form cross-links, take on the matrix structure and contain moisture therein. The type of polymer varies, some of the dressings include a thickener, and the amount of moisture varies for each product. The amount of contained moisture widely varies from 97% to about 60%. They are usually sealed with a film dressing for use. They are effective for "relatively dried open wound," i.e., a less exuding wound surface. They are also usable in an open wound that is a deep recess. When a pressure ulcer in a black stage as covered by a black crust is sealed with this covering material, autolysis is accelerated, thus facilitating debridement. When skin around a wound is fragile, sealing with a film dressing is difficult; in this case, gel is directly covered by gauze, which is effective enough.

6) Hydropolymer

Examples include, by the trade name, Tielle (manufactured by Johnson & Johnson). A hydropolymer absorption pad to be brought into contact with a wound has the characteristics that it absorbs exudate and swells toward exudate (the volume thereof increases). The main ingredient is hydrophilic polyurethane foam containing a small amount of acrylic polymer. When exudate is present, it swells toward the exudate. More specifically, when used for a recessed wound, this covering material protrudes in such a manner as to conform to the recess shape and softly fits into the wound surface. It is applicable to skin soft tissue loss wounds that are produced with post-operative wound dehiscence, flap necrosis or the like and extend broadly and deeply.

7) Silk Film

Silk fibroin, which has been used in the form of raw silk, has excellent strength, biocompatibility and biodegradablility, and silk can be used as a scaffold ingredient for encouraging regeneration of, for instance, teeth, bones, cartilages, eye tissue and blood vessels. When a silk film, nonwoven fabric or the like is used as the wound covering material in combination with the therapeutic agent for use in the treatment of a skin wound according to the invention or for use in the treatment of skin roughness according to the present disclosure, this should be effective at migration and growth of fibroblasts. A polyurethane film may be put on a surface of a silk film in order to establish a wet environment in a closed environment.

EXAMPLES

The present invention is specifically described below with reference to Examples, which by no means limit the scope of the present invention.

(Preparation Example 1)

In a reaction vessel, 500 mL of 0.08 M aqueous sodium hydrogen carbonate solution was prepared, and separately, 1,000 mL of 0.1M aqueous zinc nitrate solution was prepared as a dropping reaction solution. As a pH adjusting liquid, 30 wt% aqueous sodium hydroxide solution was prepared.
The aqueous zinc nitrate solution and the aqueous sodium hydroxide solution were added dropwise under stirring with the pH of the aqueous sodium hydrogen carbonate solution being maintained at 9.0 by means of a pH controller connected to a pump.
After all of the aqueous zinc nitrate solution was added dropwise, the reaction solution was stirred for 16 hours for conditioning.
Thereafter, the reaction solution was separated into solid and liquid fractions by centrifugation, and washing with water and centrifugation of the resulting solid were repeated three times. The precipitate thus washed was dried in vacuum, thereby obtaining hydrozincite having the composition range presented by Formula (1).

(Preparation Example 2)

Respective samples including Production Example 1 (pH: 9.0) were produced through the same process as that for Preparation Example 1 with varying pH values at synthesis from 6.0 to 10. At a pH of less than 6.5, the yield of precipitate was remarkably low because of too low a pH value.

(Preparation Example 3)

In a reaction vessel, 500 mL of 0.08 M aqueous sodium hydrogen carbonate solution was prepared, and separately, 1,000 mL of 0.1M aqueous zinc sulfate solution was prepared as a dropping reaction solution. As a pH adjusting liquid, 30 wt% aqueous sodium hydroxide solution was prepared.
The aqueous zinc sulfate solution and the aqueous sodium hydroxide solution were added dropwise under stirring with the pH of the aqueous sodium hydrogen carbonate solution being maintained at 9.0 by means of a pH controller connected to a pump.
After all of the aqueous zinc sulfate solution was added dropwise, the reaction solution was stirred for 16 hours for conditioning.
Thereafter, the reaction solution was separated into solid and liquid fractions by centrifugation, and washing with water and centrifugation of the resulting solid were repeated three times. The precipitate thus washed was dried in vacuum, thereby obtaining hydrozincite having the composition range presented by Formula (2).

(Preparation Example 4)

In a reaction vessel, 500 mL of 0.08 M aqueous sodium hydrogen carbonate solution was prepared, and separately, 1,000 mL of 0.1M aqueous zinc chloride solution was prepared as a dropping reaction solution. As a pH adjusting liquid, 30 wt% aqueous sodium hydroxide solution was prepared.
The aqueous zinc chloride solution and the aqueous sodium hydroxide solution were added dropwise under stirring with the pH of the aqueous sodium hydrogen carbonate solution being maintained at 9.0 by means of a pH controller connected to a pump.
After all of the aqueous zinc chloride solution was added dropwise, the reaction solution was stirred for 16 hours for conditioning.
Thereafter, the reaction solution was separated into solid and liquid fractions by centrifugation, and washing with water and centrifugation of the resulting solid were repeated three times. The precipitate thus washed was dried in vacuum, thereby obtaining hydrozincite having the composition range presented by Formula (3).

(Dissolution Test)

A dissolution test by a stirring method was conducted on 0.6 g each of dry powders obtained in Preparation Example 2 using 30g of saline, and the amount of dissolved $Zn^{2+}$ ions and the pH were measured. The dissolution test was conducted with hydrozincite-containing zinc carbonate hydroxide hydrate having a BET specific surface area of 10

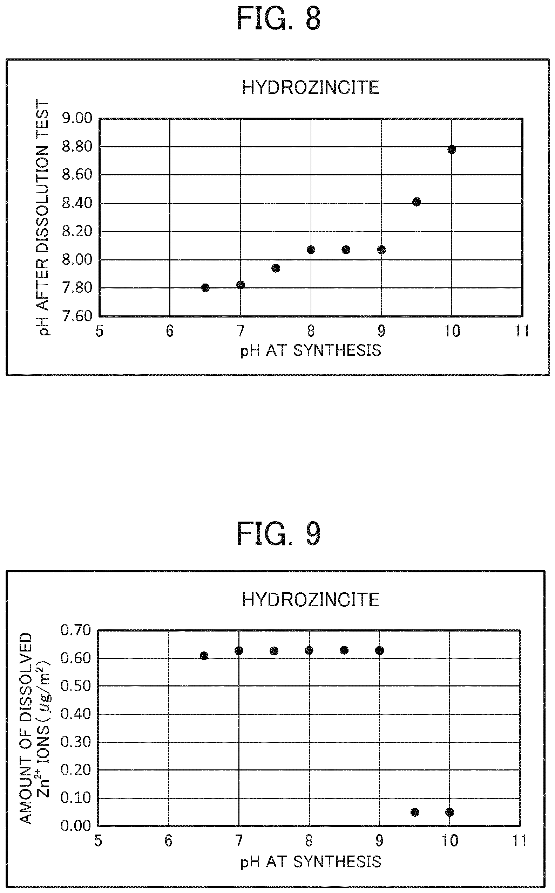

to 150 m$^2$/g, the mass ratio between the hydrozincite-containing zinc carbonate hydroxide hydrate and the saline being 1:50, and stirring being carried out at 500 rpm for 3 hours by means of a rotor. Then the pH and the Zn$^{2+}$ ion concentration after the dissolution test were measured. The results thereof are shown in FIGS. 8 and 9 and Table 2.

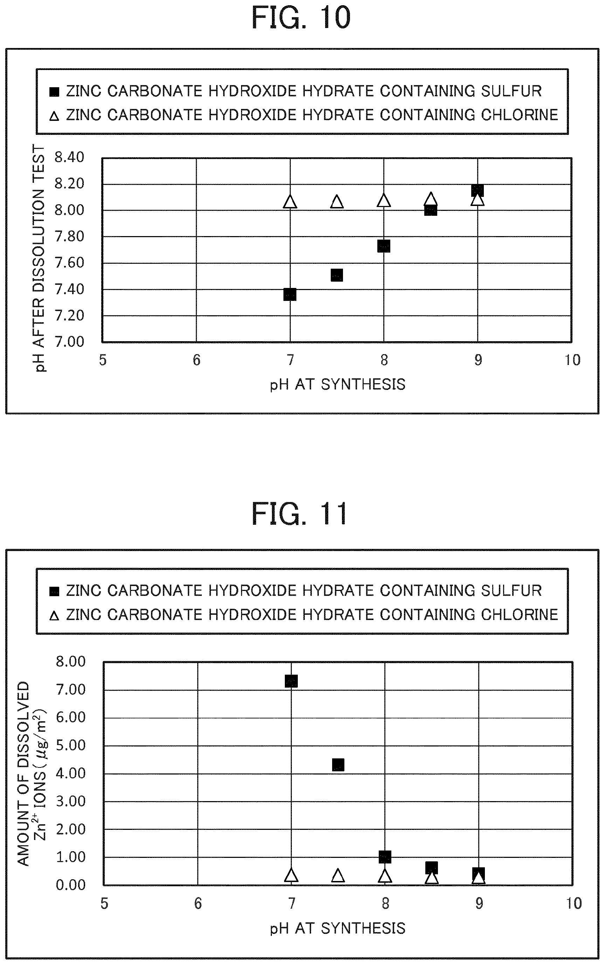

Also for zinc carbonate hydroxide hydrate including not less than 0.1 mass% but less than 1.5 mass% of sulfur as S as a result of, for instance, substitution of carbonate ions with $SO_4^{2-}$ ions and zinc carbonate hydroxide hydrate including not less than 0.05 mass% but less than 1 mass% of chlorine as Cl as a result of, for instance, substitution of carbonate ions with Cl$^-$ ions, the pH values and the Zn$^{2+}$ ion concentrations after the dissolution test made under the same conditions as above were measured. The results thereof are shown in FIGS. 10 and 11 and Tables 3 and 4.

[Table 2]

| pH at synthesis | 6 | 6.5 | 7 | 7.5 | 8 | 8.5 | 9 | 9.5 | 10 |
|---|---|---|---|---|---|---|---|---|---|
| pH after dissolution test | - | 7.80 | 7.82 | 7.94 | 8.07 | 8.07 | 8.07 | 8.41 | 8.78 |
| Amount of dissolved Zn$^{2+}$ ions after dissolution test ($\mu$g/m$^2$) | - | 0.61 | 0.63 | 0.63 | 0.63 | 0.63 | 0.63 | 0.05 | 0.05 |

[Table 3]

| pH at synthesis | 7 | 7.5 | 8 | 8.5 | 9 |
|---|---|---|---|---|---|
| pH after dissolution test | 7.36 | 7.51 | 7.73 | 8.01 | 8.15 |
| Amount of dissolved Zn$^{2+}$ ions after dissolution test ($\mu$g/m$^2$) | 7.33 | 4.32 | 1.02 | 0.63 | 0.42 |

[Table 4]

| pH at synthesis | 7 | 7.5 | 8 | 8.5 | 9 |
|---|---|---|---|---|---|
| pH after dissolution test | 8.07 | 8.07 | 8.08 | 8.09 | 8.09 |
| Amount of dissolved Zn$^{2+}$ ions after dissolution test ($\mu$g/m$^2$) | 0.38 | 0.37 | 0.35 | 0.31 | 0.30 |

For comparison, the dissolution test was conducted using reagent-grade zinc white (zinc oxide). While the amount of dissolved Zn$^{2+}$ ions was sufficient, zinc oxide has a drying effect as well as antibacterial properties and therefore is too strong for use as a therapeutic agent for skin wounds or skin roughness. In the case of using zinc hydroxide in the dissolution test, the amount of dissolution was less than 0.1 $\mu$g/m$^2$, i.e., a remarkably low value.

(Example 1)

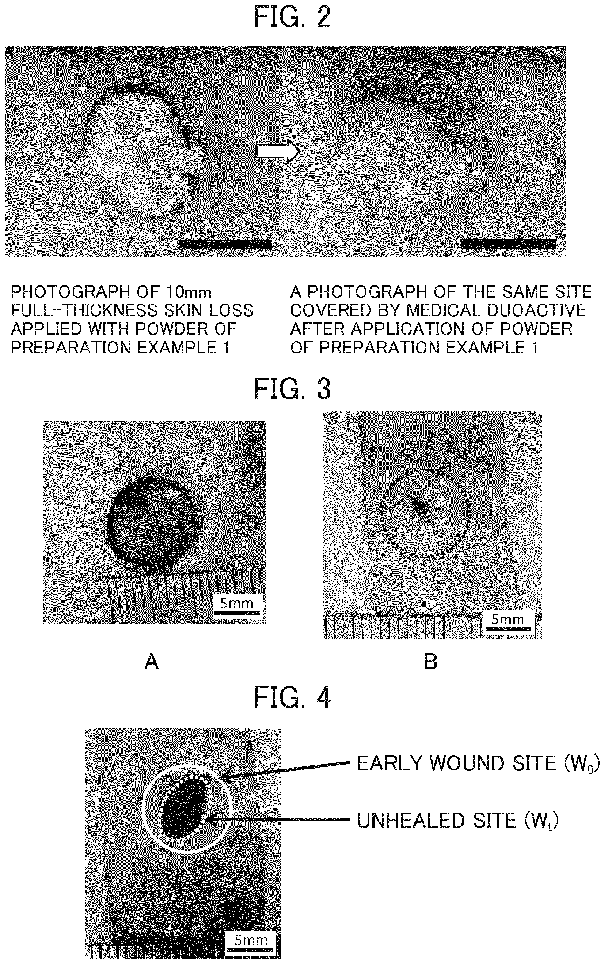

To an SD rat weighing about 500 g, 2% Sedeluck (xylazine) was administered at 0.2 mL/500 g by intramuscular injection for sedation, and general anesthesia was given by means of 2% Sevoflurane inhalation anesthetic. After local anesthesia was given by administering xylocaine (lidocaine + 2% adrenaline) to a ventral part of the rat, a full-thickness skin loss wound having a diameter of 10 mm and extending from the epidermis to the subcutaneous tissue was formed, 0.01 g of powder of Preparation Example 1 above was applied to the formed full-thickness skin loss wound (FIG. 2, left), and then the wound was covered by Duoactive being a medical wound covering material (FIG. 2, right). The wound having been just formed is shown in A of FIG. 3, and the healing condition after 2-week breeding subsequent to covering by the wound covering material is shown in B of FIG. 3. The healing condition at the wound site was observed, while the skin formation condition was observed through tissue staining.

The reepithelization rate of the wound site was measured by the method shown in FIGS. 3 and 4 and as a result was about 96%. In contrast, it was about 78% in the case of covering only with the medical wound covering material without use of Preparation Example 1. It can be said from the foregoing results that application of the therapeutic agent for skin wounds according to the invention or for skin roughness according to the present disclosure is extremely effective at wound healing.

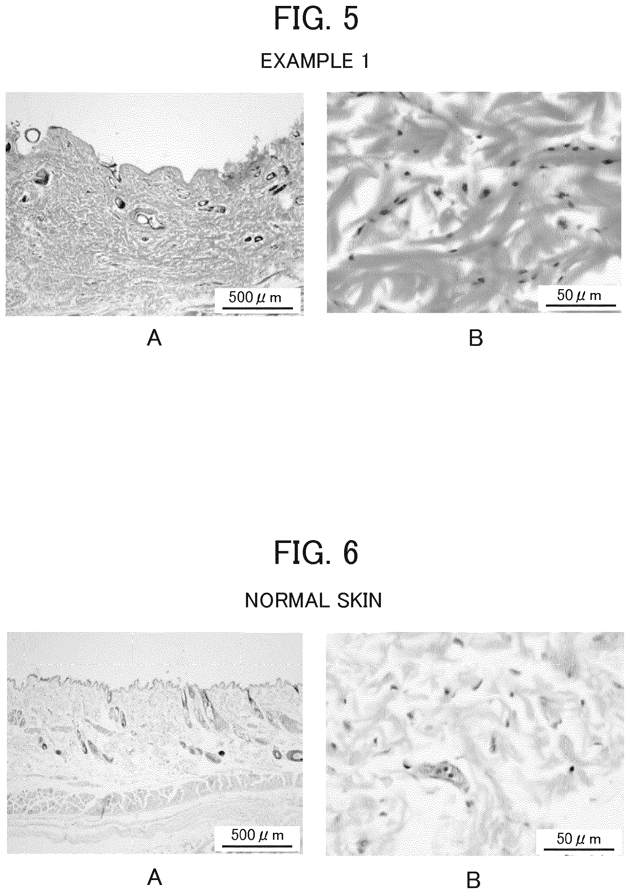

Regenerated skin tissue was excised from the rat and went through haematoxylin and eosin (H-E) staining. Hair root sites and fibroblasts were observed in autologous skin (FIG. 6), and thus the healthiness was at a high level. In the case where the powder of Preparation Example 1 was applied, as shown in FIG. 5, hair bulbs (previous stage of hair roots) and active fibroblasts were observed, while inflammatory cells, i.e., polymorphonuclear leukocytes or lymphocytes were not observed. That is, the treatment through application of the therapeutic agent for a skin wound according to the invention or for skin roughness according to the present disclosure can regenerate not only skin but also hair roots, without inflammation. The foregoing results reveal that the therapeutic agent used in the invention has a healing effect on not only skin wounds mentioned in Examples but also similar wounds resulting from severe skin roughness.

(Example 2)

Full-thickness skin loss wounds were formed in the same manner as in Example 1, applied separately with the respective powders of Preparation Example 2, and then covered by Duoactive being a medical wound covering material. The therapeutic agents used in the invention that were obtained under the production condition at a pH within the range from not less than 6.5 but less than 9.5 and that led to a reepithelization rate between 80% and 90% have excellent healing effects.

(Example 3)

Full-thickness skin loss wounds were formed in the same manner as in Example 1, applied separately with the respective powders of Preparation Examples 3 and 4, and then covered by Duoactive being a medical wound covering material. The therapeutic agents used in the invention that were obtained under the production condition at a pH within the range from not less than 6.5 but less than 9.5 and that led to a reepithelization rate between 80% and 90% have excellent healing effects.

(Example 4)

Hydrozincite having the composition range presented by Formula (1) as obtained in Preparation Example 1 and zinc oxide obtained by baking this hydrozincite at a baking temperature of 1,200°C were mixed, thereby obtaining a preparation.

(Measurement Method of Reepithelization Rate)

As shown in FIG. 3, skin was photographed in an enlarged manner at the time of wound production (A of FIG. 3) and after treatment (B of FIG. 3). The early wound site at the time of wound production is measured and drawn by a solid line on a post-treatment photograph, the area of the early wound site $W_0$ shown in FIG. 4 is measured, the area of an unhealed site (Wt) after treatment is measured using ImageJ [an open source released on the website; Wayne Rasband (NIH)], and the reepithelization rate is calculated from the measured areas using the formula below to obtain a percent value. The reepithelization rate obtained through measurement of Example 1 shown in FIGS. 3 and 4 was 95.8%.

$$\texttt{Reepithelization Rate (\%) = (W_0 - Wt) / W_0 x 100\%}$$

(Tissue Staining, Haematoxylin and Eosin (H-E) Staining)

The method of tissue staining involves, in this order, trimming (excising) a wound site; fixing in formalin; degreasing treatment (24-hour immersion in xylene); and dehydration treatment.
The dehydration treatment involves immersing in 70% ethanol for 12 hours to remove ethanol through volatilization, dehydrating in 80% ethanol, 90% ethanol and 95.5% ethanol for 30 minutes each, and washing with xylene two times.
The above was followed by, in this order, embedding treatment of a specimen in paraffin; forming a section; and H-E staining. Cell nuclei were stained in blue-violet color with haematoxylin, and the cytoplasm, collagen fibers and muscle fibers were stained in red color with eosin. Thereafter, the stained specimen is set to obtain a preparation and observed with a microscope. The observation result of Example 1 is shown in FIG. 5.
For comparison, the observation result of normal skin that was processed and stained in the same manner is shown in FIG. 6.
Table 5 below shows pH values after the dissolution test and the results of evaluation on wound healing effects (reepithelization rate, collagen regeneration, hair bulb regeneration, granulation tissue formation) after two weeks, which were obtained in Example 2. In the control, no preparation example was used, and a wound was merely covered by Duoactive being a medical wound covering material.

[Table 5]

| | 6 | 6.5 | 7 | 7.5 | 8 | 8.5 | 9 | 9.5 | 10 | - |
|---|---|---|---|---|---|---|---|---|---|---|
| pH at synthesis | 6 | 6.5 | 7 | 7.5 | 8 | 8.5 | 9 | 9.5 | 10 | - |
| pH after dissolution test | | 7.80 | 7.82 | 7.94 | 8.07 | 8.07 | 8.07 | 8.41 | 8.78 | - |
| Amount of dissolved $Zn^{2+}$ ions after dissolution test ($\mu g/m^2$) | - | 0.61 | 0.63 | 0.63 | 0.63 | 0.63 | 0.63 | 0.05 | 0.05 | - |
| Healing condition after 2 weeks | | | | | | | | | | Control |
| Degree of reepithelization | - | 1.1 | 1.1 | 1.6 | 1.4 | 1.3 | 1.4 | - | - | 1 |
| Thickness of collagen | - | Good | Good | Good | Good | Good | Good | - | - | Average |
| Orientation of collagen | - | Good | Good | Good | Good | Good | Good | - | - | Poor |
| Hair bulb | - | Good | Good | Average | Average | Average | Poor | - | - | Poor |
| Granulation tissue formation | | Good | Good | Good | Good | Good | Good | - | - | Good |

Tables 6 and 7 below show pH values after the dissolution test and the results of evaluation on wound healing effects (reepithelization rate, collagen regeneration, hair bulb regeneration, granulation tissue formation) after two weeks, which were obtained in Examples 3 and 4, respectively.

[Table 6]

| pH at synthesis | 7 | 7.5 | 8 | 8.5 | 9 | - |
|---|---|---|---|---|---|---|
| pH after dissolution test | 7.36 | 7.51 | 7.73 | 8.01 | 8.15 | - |
| Amount of dissolved $Zn^{2+}$ ions after dissolution test ( $\mu g/m^2$) | 7.33 | 4.32 | 1.02 | 0.63 | 0.42 | - |
| Healing condition after 2 weeks | | | | | | Control |
| Degree of reepithelization | 1.1 | 1.08 | 1.05 | 1 | 0.95 | 1 |
| Thickness of collagen | Good | Good | Good | Good | Average | Average |
| Orientation of collagen | Good | Good | Average | Average | Average | Poor |
| Hair bulb | Average | Average | Average | Poor | Poor | Poor |
| Granulation tissue formation | Good | Good | Good | Good | Good | Good |

[Table 7]

| pH at synthesis | 7 | 7.5 | 8 | 8.5 | 9 | - |
|---|---|---|---|---|---|---|
| pH after dissolution test | 8.07 | 8.07 | 8.08 | 8.09 | 8.09 | - |
| Amount of dissolved $Zn^{2+}$ ions after dissolution test ( $\mu g/m^2$) | 0.38 | 0.37 | 0.35 | 0.31 | 0.30 | - |
| Healing condition after 2 weeks | | | | | | Control |
| Degree of reepithelization | 1.05 | 1.05 | 1.02 | 1.02 | 1.01 | 1 |
| Thickness of collagen | Good | Good | Good | Good | Good | Average |
| Orientation of collagen | Good | Good | Good | Good | Average | Poor |
| Hair bulb | Average | Average | Poor | Poor | Poor | Poor |
| Granulation tissue formation | Good | Good | Good | Good | Good | Good |

For comparison, the results of the control are shown together in Tables 5 to 7. A clear effect of hydrozincite used in the invention is recognizable in regenerated tissue, especially in terms of the appearance of collagen.

Thus, in this invention, an inorganic material that is the therapeutic agent used in the invention is used with an organic material that is a commercial wound covering material, whereby the organic and inorganic materials are hybridized, which produces a great synergy and greatly improves a wound healing ability.

Methods of evaluations shown in Tables 5 to 7 are as follows: The reepithelization rate was evaluated by the foregoing measurement method with taking the control as 1; For collagen, the state where thick collagen fibers extend in one direction was observed; For a hair bulb, observation results are given on whether a hair bulb is recognizable; For granulation tissue formation, evaluations are given on whether tissue composed of capillaries and fibroblasts is observable.

Collagen: For collagen, the thickness and orientation of collagen fibers were evaluated as compared to a healthy skin cell.

Collagen thickness:

Poor; Considerably thin
Average; Thin
Good; Slightly thinner
Excellent; Almost same degree

Collagen orientation:

Poor; Disordered
Average; Considerably inferior
Good; Slightly inferior
Excellent; Almost same degree

Hair bulb:

Poor; No hair bulb is recognized.
Average; Hair bulb formation is recognized.
Good; Hair bulbs are formed.
Excellent; Hair bulb formation is notably recognized.

Granulation tissue formation:

Poor; No granulation tissue is recognized.
Good; Granulation tissue is recognized.
Excellent; Granulation tissue is notably recognized.

Industrial Applicability

The therapeutic agent used in the invention is effective at treating skin wounds or skin roughness extending from the epidermis to the dermis. Thus, it has a treatment effect on not only severe wounds or skin roughness but also various large and small wounds and skin roughness, and can be widely used as a vulnerary.

REFERENCE SIGNS LIST

1 a skin wound or rough skin part extending from the epidermis to the dermis

**Claims**

1. A therapeutic agent for use in the treatment of a skin wound,

    wherein after a dissolution test, by a stirring method, of at least one hydrozincite-containing zinc carbonate hydroxide hydrate selected from the group consisting of hydrozincite-containing zinc carbonate hydroxide hydrate, zinc carbonate hydroxide hydrate including not less than 0.1 mass% but less than 1.5 mass% of sulfur as S as a result of at least partial substitution of carbonate ions with $SO_4^{2-}$ ions, and zinc carbonate hydroxide hydrate including not less than 0.05 mass% but less than 1 mass% of chlorine as Cl as a result of at least partial substitution of carbonate ions with $Cl^-$ ions, an amount of dissolved $Zn^{2+}$ ions is not less than 0.1 $\mu g/m^2$ and pH is not less than 7.2 but less than 8.3,
    where the dissolution test is conducted with the hydrozincite-containing zinc carbonate hydroxide hydrate having a BET specific surface area of 10 to 150 $m^2/g$, a mass ratio between the hydrozincite-containing zinc carbonate hydroxide hydrate and saline being 1:50, and stirring being carried out at 500 rpm at 37°C for 3 hours by means of a rotor.

2. The therapeutic agent for use in the treatment of a skin wound according to claim 1,
    wherein the hydrozincite-containing zinc carbonate hydroxide hydrate is expressed by Formula (1):

$$Zn_{4-6}(CO_3)_{1-3}(OH)_{5-6} \cdot nH_2O \qquad (1)$$

    where n is 0 to 6,
    and a molar ratio between Zn and $CO_3$ is $Zn/CO_3 = 2.5$ to 3.3.

3. The therapeutic agent for use in the treatment of a skin wound according to claim 1,
    wherein the zinc carbonate hydroxide hydrate containing hydrozincite and not less than 0.1 mass% but less than 1.5 mass% of sulfur as S is expressed by Formula (2):

$$Zn_{4-6}((1 - x)CO_3 + x(SO_4))_{1-3}(OH)_{5-6} \cdot nH_2O \qquad (2)$$

where n is 0 to 6 and x is 0.005 to 0.1,
and a molar ratio between Zn and $((1 - x)CO_3 + x(SO_4))$ is $Zn/((1 - x)CO_3 + x(SO_4)) = 2.5$ to $3.3$.

4. The therapeutic agent for use in the treatment of a skin wound according to claim 1,
wherein the zinc carbonate hydroxide hydrate containing hydrozincite and not less than 0.05 mass% but less than 1 mass% of chlorine as Cl is expressed by Formula (3):

$$Zn_{4-6}((1 - x)CO_3 + xCl)_{1-3}(OH)_{5-6} \cdot nH_2O \qquad (3)$$

where n is 0 to 6 and x is 0.005 to 0.1,
and a molar ratio between Zn and $((1 - x)CO_3 + xCl)$ is $Zn/((1 - x)CO_3 + xCl) = 2.5$ to $3.3$.

5. The therapeutic agent for use in the treatment of a skin wound according to any one of claims 1 to 4,
wherein in a pattern of XRD diffraction peaks of the hydrozincite-containing zinc carbonate hydroxide hydrate, the zinc carbonate hydroxide hydrate including not less than 0.1 mass% but less than 1.5 mass% of sulfur as S as a result of at least partial substitution of carbonate ions with $SO_4^{2-}$ ions, or the zinc carbonate hydroxide hydrate including not less than 0.05 mass% but less than 1 mass% of chlorine as Cl as a result of at least partial substitution of carbonate ions with $Cl^-$ ions as expressed by a composition formula according to any one of claims 2 to 4, a structure $Zn_5(CO_3)_2OH)_6 \cdot nH_2O$, $Zn_5((1 - x)CO_3 + x(SO_4))_2(OH)_6 \cdot nH_2O$ or $Zn_5((1 - x)CO_3 + xCl)_2(OH)_6 \cdot nH_2O$ [where x is 0.005 to 0.1 and n is 0 to 6 in each chemical formula] is dominant in a corresponding substance, with an a axis being 13.3 to 13.8, a b axis being 6.2 to 6.4, a c axis being 5.25 to 5.5, and $\beta$ being 94.9 to 97.5.

6. The therapeutic agent for use in the treatment of a skin wound according to any one of claims 2 to 5,
wherein when the therapeutic agent according to any one of claims 2 to 5 is dissolved in saline for use with a ratio between the therapeutic agent and the saline being 0.1 g/L to 100 g/L, with the therapeutic agent being expressed by one of Formulae (1) to (3) and n = 0 (anhydrous), a zinc concentration is 45 mass% to 75 mass% in terms of zinc metal with respect to a total amount of the therapeutic agent, and a zinc concentration of the therapeutic agent in the saline is 0.045 g/L to 75 g/L.

7. The therapeutic agent for use in the treatment of a skin wound according to any one of claims 1 to 6,
wherein the skin wound is a skin wound extending from epidermis to dermis.

8. A medical device for treating a skin wound, comprising: the therapeutic agent according to any one of claims 1 to 7 that is applied to a skin wound; and a wound covering material that retains the skin wound in a closed environment.

9. The medical device for treating a skin wound according to claim 8,
wherein the wound covering material is at least one selected from the group consisting of a polyurethane film dressing, a hydrocolloid dressing, a polyurethane foam dressing, an alginate covering material, a hydrogel dressing, a hydropolymer, a cellulose film and a silk film.

10. The medical device for treating a skin wound according to claim 8 or 9,
wherein the therapeutic agent for use in the treatment of a skin wound according to any one of claims 1 to 7 is applied, contained or attached on or in the wound covering material.

11. A medical set for treating a skin wound, comprising the therapeutic agent according to any one of claims 1 to 7 and a wound covering material in combination.

**Patentansprüche**

1. Therapeutisches Mittel zur Verwendung bei der Behandlung einer Hautwunde,

wobei nach einem Auflösungstest durch ein Rührverfahren von mindestens einem hydrozinkithaltigen Zinkcarbonathydroxidhydrat, ausgewählt aus der Gruppe, bestehend aus hydrozinkithaltigem Zinkcarbonathydroxidhydrat, Zinkcarbonathydroxidhydrat, das nicht weniger als 0,1 Massen-%, aber weniger als 1,5 Massen-% Schwefel als S als Ergebnis einer zumindest teilweisen Substitution von Carbonationen durch $SO_4^{2-}$-Ionen enthält, und Zinkcarbonathydroxidhydrat, das nicht weniger als 0,05 Massen-%, aber weniger als 1 Massen-% Chlor als Cl als Ergebnis einer zumindest teilweisen Substitution von Carbonationen durch $Cl^-$-Ionen enthält, eine Menge an gelösten $Zn^{2+}$-Ionen nicht weniger als 0,1 $\mu g/m^2$ beträgt und der pH-Wert nicht weniger als 7,2, aber weniger als 8,3 beträgt,
wobei der Auflösungstest mit dem hydrozinkithaltigen Zinkcarbonathydroxidhydrat mit einer spezifischen BET-Oberfläche von 10 bis 150 $m^2/g$ durchgeführt wird, wobei das Massenverhältnis zwischen dem hydrozinkithaltigen Zinkcarbonathydroxidhydrat und der Salzlösung 1:50 beträgt und das Rühren bei 500 U/min bei 37°C für 3 Stunden mittels eines Rotors durchgeführt wird.

2. Das therapeutische Mittel zur Verwendung bei der Behandlung einer Hautwunde nach Anspruch 1, wobei das hydrozinkithaltige Zinkcarbonathydroxidhydrat durch die Formel (1) ausgedrückt wird:

$$Zn_{4-6}(CO_3)_{1-3}(OH)_{5-6} \cdot nH_2O \qquad (1)$$

wobei n 0 bis 6 ist,
und das Molverhältnis zwischen Zn und $CO_3$ $Zn/CO_3$ = 2,5 bis 3,3 ist.

3. Das therapeutische Mittel zur Verwendung bei der Behandlung einer Hautwunde nach Anspruch 1, wobei das Zinkcarbonathydroxidhydrat, das Hydrozinkit und nicht weniger als 0,1 Massen-%, aber weniger als 1,5 Massen-% Schwefel als S enthält, durch die Formel (2) ausgedrückt wird:

$$Zn_{4-6}((1 - x)CO_3 + x(SO_4))_{1-3}(OH)_{5-6} \cdot nH_2O \qquad (2)$$

wobei n 0 bis 6 ist und x 0,005 bis 0,1 ist,
und das Molverhältnis zwischen Zn und $((1 - x)CO_3 + x(SO_4))$ $Zn/((1 - x)CO_3 + x(SO_4))$ = 2,5 bis 3,3 ist.

4. Das therapeutische Mittel zur Verwendung bei der Behandlung einer Hautwunde nach Anspruch 1, wobei das Zinkcarbonathydroxidhydrat, das Hydrozinkit und nicht weniger als 0,05 Massen-%, aber weniger als 1 Massen-% Chlor als Cl enthält, durch die Formel (3) ausgedrückt wird:

$$Zn_{4-6}((1 - x)CO_3 + xCl)_{1-3}(OH)_{5-6} \cdot nH_2O \qquad (3)$$

wobei n 0 bis 6 ist und x 0,005 bis 0,1 ist,
und das Molverhältnis zwischen Zn und $((1 - x)CO_3 + xCl)$ $Zn/((1 - x)CO_3 + xCl)$ = 2,5 bis 3,3 ist.

5. Das therapeutische Mittel zur Verwendung bei der Behandlung einer Hautwunde nach einem der Ansprüche 1 bis 4, wobei in einem Muster von XRD-Beugungspeaks des hydrozinkithaltigen Zinkcarbonathydroxidhydrats, des Zinkcarbonathydroxidhydrats, das nicht weniger als 0,1 Massen-%, aber weniger als 1,5 Massen-% Schwefel als S als Ergebnis einer zumindest teilweisen Substitution von Carbonationen durch $SO_4^{2-}$-Ionen enthält, oder des Zinkcarbonathydroxidhydrats, das nicht weniger als 0,05 Massen-%, aber weniger als 1 Massen-% Chlor als Cl als Ergebnis einer zumindest teilweisen Substitution von Carbonationen durch $Cl^-$-Ionen enthält, ausgedrückt durch eine Zusammensetzungsformel nach einem der Ansprüche 2 bis 4, eine Struktur $Zn_5(CO_3)_2(OH)_6 \cdot nH_2O$, $Zn_5((1 - x)CO_3 + x(SO_4))_2(OH)_6 \cdot nH_2O$ oder $Zn_5((1 - x)CO_3 + xCl)_2(OH)_6 \cdot nH_2O$ [wobei x 0,005 bis 0,1 ist und n 0 bis 6 ist in jeder chemischen Formel] in einer entsprechenden Substanz dominiert, wobei eine *a*-Achse 13,3 bis 13,8, eine *b*-Achse 6,2 bis 6,4, eine c-Achse 5,25 bis 5,5 und $\beta$ 94,9 bis 97,5 beträgt.

6. Das therapeutische Mittel zur Verwendung bei der Behandlung einer Hautwunde nach einem der Ansprüche 2 bis 5, wobei, wenn das therapeutische Mittel nach einem der Ansprüche 2 bis 5 zur Verwendung in Salzlösung gelöst wird, wobei das Verhältnis zwischen dem therapeutischen Mittel und der Salzlösung 0,1 g/L bis 100 g/L beträgt, wobei das therapeutische Mittel durch eine der Formeln (1) bis (3) ausgedrückt wird und n = 0 (wasserfrei) ist, die Zinkkonzentration 45 Massen-% bis 75 Massen-%, ausgedrückt als Zinkmetall, in Bezug auf die Gesamtmenge des therapeutischen Mittels beträgt, und die Zinkkonzentration des therapeutischen Mittels in der Salzlösung 0,045 g/L bis 75 g/L beträgt.

7. Das therapeutische Mittel zur Verwendung bei der Behandlung einer Hautwunde nach einem der Ansprüche 1 bis 6, wobei die Hautwunde eine Hautwunde ist, die sich von der Epidermis zur Dermis erstreckt.

8. Medizinische Vorrichtung zur Behandlung einer Hautwunde, umfassend: das therapeutische Mittel nach einem der Ansprüche 1 bis 7, das auf eine Hautwunde aufgetragen wird; und ein Wundabdeckmaterial, das die Hautwunde in einer geschlossenen Umgebung hält.

9.  Die medizinische Vorrichtung zur Behandlung einer Hautwunde nach Anspruch 8,
    wobei das Wundabdeckmaterial mindestens eines ist, ausgewählt aus der Gruppe, bestehend aus einem Polyurethanfilmverband, einem Hydrokolloidverband, einem Polyurethanschaumverband, einem Alginatabdeckmaterial,
    einem Hydrogelverband, einem Hydropolymer, einem Zellulosefilm und einem Seidenfilm.

10. Die medizinische Vorrichtung zur Behandlung einer Hautwunde nach Anspruch 8 oder 9,
    wobei das therapeutische Mittel zur Verwendung bei der Behandlung einer Hautwunde nach einem der Ansprüche
    1 bis 7 auf oder in dem Wundabdeckmaterial aufgebracht, enthalten oder befestigt ist.

11. Medizinisches Set zur Behandlung einer Hautwunde, umfassend das therapeutische Mittel nach einem der Ansprüche 1 bis 7 und ein Wundabdeckmaterial in Kombination.

**Revendications**

1.  Agent thérapeutique pour l'utilisation dans le traitement d'une lésion cutanée,

    après un test de dissolution, par un procédé d'agitation, d'au moins un hydroxyde de carbonate de zinc hydraté
    contenant de l'hydrozincite sélectionné dans le groupe constitué de l'hydroxyde de carbonate de zinc hydraté
    contenant de l'hydrozincite, l'hydroxyde de carbonate de zinc hydraté comprenant pas moins de 0,1 % en masse
    mais moins de 1,5 % en masse de soufre comme S en résultat d'une substitution au moins partielle des ions
    carbonates par des ions $SO_4^{2-}$, et de l'hydroxyde de carbonate de zinc hydraté comprenant pas moins de 0,05
    % en masse mais moins de 1 % en masse de chlore comme Cl en résultat d'une substitution au moins partielle
    des ions carbonates avec des ions $Cl^-$, une quantité des ions $Zn^{2+}$ dissous n'étant pas inférieure à 0,1 $\mu$g/m²
    et le pH n'étant pas inférieur à 7,2 mais inférieur à 8,3,
    où le test de dissolution est conduit avec l'hydroxyde de carbonate de zinc hydraté contenant de l'hydrozincite
    ayant une surface spécifique B.E.T. de 10 à 150 m²/g, un rapport en masse entre l'hydroxyde de carbonate de
    zinc hydraté contenant de l'hydrozincite et le composé salin étant de 1:50, et l'agitation étant effectuée à 500
    tr/min à 37°C durant 3 heures à l'aide d'un rotor.

2.  Agent thérapeutique pour l'utilisation dans le traitement d'une lésion cutanée selon la revendication 1,

    l'hydroxyde de carbonate de zinc hydraté contenant de l'hydrozincite étant exprimé par la Formule (1) :

    $$Zn_{4-6}(CO_3)_{1-3}(OH)_{5-6} \cdot nH_2O \qquad (1)$$

    où n a la valeur de 0 à 6,
    et un rapport molaire compris entre Zn et $CO_3$ étant $Zn/CO_3$ = 2, 5 à 3,3.

3.  Agent thérapeutique pour l'utilisation dans le traitement d'une lésion cutanée selon la revendication 1,

    l'hydroxyde de carbonate de zinc hydraté contenant de l'hydrozincite et pas moins de 0,1 % en masse mais
    moins de 1,5 % en masse de soufre comme S étant exprimé par la Formule (2) :

    $$Zn_{4-6}((1-x)CO_3 + x(SO_4))_{1-3}(OH)_{5-6} \cdot nH_2O \qquad (2)$$

    où n a la valeur de 0 à 6 et x a la valeur de 0,005 à 0,1,
    et un rapport molaire compris entre Zn et $((1-x)CO_3 + x(SO_4))$ étant $Zn/((1-x)CO_3 + x(SO_4))$ = 2,5 à 3,3.

4.  Agent thérapeutique pour l'utilisation dans le traitement d'une lésion cutanée selon la revendication 1,

    l'hydroxyde de carbonate de zinc hydraté contenant de l'hydrozincite et pas moins de 0,05 % en masse mais
    moins de 1 % en masse de chlore comme Cl étant exprimé par la Formule (3) :

    $$Zn_{4-6}((1-x)CO_3 + xCl)_{1-3}(OH)_{5-6} \cdot nH_2O \qquad (3)$$

où n a la valeur de 0 à 6 et x a la valeur de 0,005 à 0,1,
et un rapport molaire compris entre Zn et $((1 - x)CO_3 + xCl)$ étant $Zn/((1 - x)CO_3 + xCl)$ = 2, 5 à 3,3.

5. Agent thérapeutique pour l'utilisation dans le traitement d'une lésion cutanée selon l'une quelconque des revendications 1 à 4,
dans un profil des pics de diffraction XRD de l'hydroxyde de carbonate de zinc hydraté contenant de l'hydrozincite, l'hydroxyde de carbonate de zinc hydraté comprenant pas moins de 0, 1 % en masse mais moins de 1,5 % en masse de soufre comme S en résultat d'une substitution au moins partielle des ions carbonates avec des ions $SO_4^{2-}$, ou l'hydroxyde de carbonate de zinc hydraté comprenant pas moins de 0,05 % en masse mais moins de 1 % en masse de chlore comme Cl en résultat d'une substitution au moins partielle des ions carbonates avec des ions Cl⁻ tel qu'exprimé par une formule de composition selon l'une quelconque des revendications 2 à 4, une structure $Zn_5(CO_3)_2(OH)_6 \cdot nH_2O$, $Zn_5((1 - x)CO_3 + x(SO_4))_2(OH)_6 \cdot nH_2O$ ou $Zn_5((1 - x)CO_3 + xCl)_2(OH)_6 \cdot nH_2O$ [où x a la valeur de 0, 005 à 0, 1 et n a la valeur de 0 à 6 dans chaque formule chimique] étant dominant dans une substance correspondante, avec un axe a étant de 13.3 à 13.8, un axe b étant de 6.2 à 6.4, un axe c étant de 5.25 à 5.5, et $\beta$ étant de 94.9 à 97.5.

6. Agent thérapeutique pour l'utilisation dans le traitement d'une lésion cutanée selon l'une quelconque des revendications 2 à 5,
lorsque l'agent thérapeutique selon l'une quelconque des revendications 2 à 5 est dissous dans un composé salin pour l'utilisation sous un rapport compris entre l'agent thérapeutique et le composé salin étant de 0,1 g/l à 100 g/l, avec l'agent thérapeutique étant exprimé par l'une des Formules (1) à (3) et n = 0 (anhydre), une concentration en zinc étant de 45 % en masse à 75 % en masse en termes de métal zinc par rapport à une quantité totale de l'agent thérapeutique, et une concentration en zinc de l'agent thérapeutique dans le composé salin étant de 0,045 g/l à 75 g/l.

7. Agent thérapeutique pour l'utilisation dans le traitement d'une lésion cutanée selon l'une quelconque des revendications 1 à 6,
la lésion cutanée étant une lésion cutanée s'étendant de l'épiderme au derme.

8. Dispositif médical de traitement d'une lésion cutanée, comprenant : l'agent thérapeutique selon l'une quelconque des revendications 1 à 7 qui est appliqué à une lésion cutanée ; et un matériau de recouvrement de lésion qui retient la lésion cutanée dans un environnement fermé.

9. Dispositif médical de traitement d'une lésion cutanée selon la revendication 8,
le matériau de recouvrement de lésion étant au moins l'un sélectionné dans le groupe constitué d'un pansement de film de polyuréthane, d'un pansement d'hydrocolloïde, d'un pansement de mousse de polyuréthane, d'un matériau de recouvrement en alginate, d'un pansement hydrogel, d'un hydropolymère, d'un film de cellulose et d'un film de soie.

10. Dispositif médical de traitement d'une lésion cutanée selon la revendication 8 ou 9,
l'agent thérapeutique pour l'utilisation dans le traitement d'une lésion cutanée selon l'une quelconque des revendications 1 à 7 étant appliqué, contenu ou fixé sur ou dans le matériau de recouvrement de lésion.

11. Ensemble médical de traitement d'une lésion cutanée, comprenant l'agent thérapeutique selon l'une quelconque des revendications 1 à 7 et un matériau de recouvrement de lésion en combinaison.

FIG. 1

EP 3 308 794 B1

## FIG. 2

PHOTOGRAPH OF 10mm
FULL-THICKNESS SKIN LOSS
APPLIED WITH POWDER OF
PREPARATION EXAMPLE 1

A PHOTOGRAPH OF THE SAME SITE
COVERED BY MEDICAL DUOACTIVE
AFTER APPLICATION OF POWDER
OF PREPARATION EXAMPLE 1

## FIG. 3

A

B

## FIG. 4

EARLY WOUND SITE ($W_0$)

UNHEALED SITE ($W_t$)

# FIG. 5

EXAMPLE 1

500 μm

A

50 μm

B

# FIG. 6

NORMAL SKIN

500 μm

A

50 μm

B

# FIG. 7

EPIDERMIS
DERMIS
FAT LAYER

A

IN DRY
ENVIRONMENT

CRUST

EPIDERMAL
CELL

B

SCAR
TISSUE

FIBROBLAST

C

IN WET
ENVIRONMENT

COVERING
MATERIAL

EPIDERMAL
CELL

D

E

EP 3 308 794 B1

# FIG. 8

# FIG. 9

# FIG. 10

# FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004161684 A
- JP 2005006995 A
- WO 2009129358 A2
- WO 9625913 A1
- US 2014134272 A1
- US 2011159106 A1
- WO 2014026161 A1
- JP 2004269372 A

- WO 03082229 A1
- JP 2005515191 A
- JP 2004534560 A
- JP 2014511851 A
- JP 2005524690 A
- CN 103622885 A
- CN 104546629 A

**Non-patent literature cited in the description**

- **KEN-ICHI SHOFUDA ; KAORU MIYAZAKI.** *Kagaku to seibutsu [Chemistry and biology,* 1997, vol. 35 (12

- **KRÜGER et al.** *Journal of Orthopaedic Surgery and Research,* 2012, vol. 7, 10